# EUROPEAN PATENT APPLICATION

(11) **EP 1 964 555 A1**
(43) Date of publication of application: **03.09.2008**
(21) Application number: 06783800.3
(22) Date of filing: 12.07.2006
(51) Int. Cl.: A61K 31/205, A61K 31/137, A61P 3/04

(54) **PHARMACEUTICAL COMPOSITIONS CONTAINING SUBSTANCES THAT INHIBIT THE REUPTAKE OF NORADRENALINE, DOPAMINE AND SEROTONIN, WHICH ARE COMBINED WITH AN AMINO ACID FOR THE TREATMENT OF OBESITY AND OVERWEIGHT**

(30) Priority: 20.12.2005 MX PA05014089
(71) Applicant: Espinosa Abdala, Leopoldo de Jesús, C.P. 45110 Zapopan, Jalisco , México (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco, México (MX); ÁLVAREZ OCHOA, Victor Guillermo, C.P. 45222 Zapopan, Jalisco, México (MX); SANTOS MURILLO, Josefina, C.P. 44600 Zapopan, Jalisco, México (MX); GARCÍA ARMENTA, Patricia, C.P. 45110 Zapopan, Jalisco, México (MX); ESPINOSA ABDALA, Leopoldo de Jesus, C.P. 45110 Zapopan, Jalisco (MX)
(74) Representative: Carvajal y Urquijo, Isabel
(86) International application number: PCT/MX2006/000070
(87) International publication number: WO 2007/073133

(57) **Abstract**

The invention relates to the pharmaceutical industry in general and to the pharmaceutical industry involved in the production of medicaments for controlling obesity and overweight. More specifically, the invention relates to a composition containing a substance that inhibits the reuptake of noradrenalin, dopamine and serotonin and an amino acid. The composition is **characterized in that** the aforementioned combination or association consists of a selected substance that inhibits the reuptake of noradrenalin, dopamine and serotonin, known as Sibutramine, and an amino acid known as L-carnitine, in order to produce a product which has a synergistic effect for the treatment of obesity and overweight, with fewer side effects.

## Description

### FIELD OF THE INVENTION

The present invention generally relates to pharmaceutical industry and in particular to drug preparer pharmaceutical industry for controlling obesity and overweight.

### BACKGROUND OF THE INVENTION

Obesity has become one of the major threatens to public health due to the increasing incidence thereof and its capacity to induce chronic pathologies in almost all the organism systems. During the last fifteen years advances in the understanding of such disease have been achieved which go far beyond those achieved during the previous one hundred years; this has created a rational basis for new therapies able to prevent or revert obesity.

Obesity is one the pathologies in whose understanding the most rapid advances in the last years have been achieved and one of the most frequent comorbilities in patients of any general or specialist physician.

Obesity is an abnormal or excessive fat accumulation in the adipose tissue, "in such an extent that health becomes deteriorated", the World Health Organization (WHO)₁ affirms.

This conceptual definition of obesity implies certain difficulties to change it into an operative definition. How much deterioration is it necessary in order for it to be considered significant in health terms? That adipose accumulation extent able to make health become deteriorated, is the same for all the individuals?, and if it is not, what factors modify the threshold from which adiposity affects health?:

That difficulty in homogenizing criteria makes difficult to compare the incidence and prevalence rates among different populations or to provide a global consolidation. Additionally, obesity prevalence is increased with age, and the etarean distribution of the population is different from country to country, thus the rates adjusted by age should always be compared.

In spite of all the restrictions in quantifying overweight load in the society, there is a consensus at one point: obesity is the great epidemic of the XXI century.

In the United Estates, the first economic power in present times, the situation is constituted by an imminent warning to the nations worldwide: 65% of the adult individuals present obesity to some extent, a body mass index higher than 25 kg/m² (BMI > 25 kg/m²) and what is worse, 30.5% of the adults are obese, BMI> 30 kg/m².

Due to its magnitude and transcendence, it is considered as a Public Health problem in Mexico, as shown by that reported by 2000 Mexican National Health Survey, that from early ages there is a high prevalence of overweight and obesity as a 29% of overweight and obesity is present in adolescents and as to adults and women ranging from 20-59 years old a 36.1% of overweight and 28.1% of obesity was present and in men from the same etarean group 40.9% of overweight and 18.6 of obesity.

What is the cause for this epidemic? The two determinants for an individual's weight are the particular genetic load thereof and the environment to which the individual is exposed.

As it is in the last 30 years that obesity epidemic has been generated, one of the two factors should have notoriously been modified during that period. Changes in the population or species genome, no matter how small they are, are produced along quite expanded periods; a modification in the genome is therefore unlikely to have been occurred in just 30 years.

The whole massive industrialization process, especially in transportation, telecommunication and microelectronics, took place during the second half of the XX century, so the main suspect as the responsible for the acute increase in obesity and overweight prevalence is the environment.

The environmental elements which determine the body weight are essentially caloric intake and physical activity level. Caloric intake has indeed been modified in the last thirty years; although not enough to explain the epidemic, it did occur in a constant fashion and in most of the populations. The causes for a higher caloric intake *per capital* are: Higher energy availability *per se,* it is necessary to make a quite less effort to access foods providing the body with calories; a higher number of meals consumed outside: this makes the consumption of energetically denser, fiber and water poor foods to be increased.

Higher consumption of gaseous beverages: most people do not consider "taking a soda" as a caloric intake and many children and adults might even drink 4-5 gaseous beverages in a single day (each non-dietetic soda contains about 140 calories). An increase in the serving size: although the diet composition is qualitatively similar, the servings' average size, particularly from food sources of complex carbohydrates, has been increased.

The second environmental factor having a decisive influence on the body weight regulation is the physical activity level, being perhaps the one which has more drastically been modified at the end of the XX century.

From the above-mentioned, it can be concluded that the true seeds of the obesity epidemic is in the daily life, in the occidental society lifestyle, promoted as successful and worthy to be imitated by the media.

The really worrying about this epidemic are the consequences resulting in both the individual and the society.

*Cardiovascular disease:* Obesity must be considered a major risk factor for cardiovascular disease.

*Cerebrovascular disease:* The risk of ischemic cerebrovascular accident (CVA) is increased with the increase of the body mass index (BMI) and the Waist-Hip Ratio (WHR) in both men and women.

*Type-II Diabetes Mellitus* 80% of diabetes risk on the population can be attributable to the combination of overweight and physical inactivity.

Not only the BMI, but also various adiposity indicators, are correlated with the risk of de developing diabetes mellitus: weight gain on adulthood, waist perimeter (WP), waist-hip rate (WHR).

*Cancer:* according to the second cancer prevention study from the United States, which included less than a million people observed for 16 years, the excess of body weigh increased the risk of dying from at least 15 different cancers: esophagus cancer, stomach cancer, colorectal cancer, liver cancer, gallbladder cancer, pancreas cancer, prostate cancer, kidney cancer, non-Hodgkin's lymphoma, multiple myeloma, breast cancer, uterus cancer (non-cervical), cerviz cancer and ovary cancer.

*Osteoarthritis:* Overweight and obesity noticeably increase OA risk, particularly in hip and knees, which though not affecting mortality figures, cause severe pain and disability, considerably deteriorating the quality of life.

*Other pathologies:* Overweight also causes or fosters the appearance of various pathologies, among them: sleep apnea, asthma, cataracts, benign prostate hypertrophy, menstrual irregularities, pregnancy complications and depression.

*Impact over quality of life:* the obese patients are discriminated and socially rejected, they have difficulties to get a well-paid job, their life and health insurance policy are more expensive, they have less vitality and, in most of the cases, they are the butt of everyone's jokes and subject to humiliation.

The fine mechanisms which regulate the body weight oscillations virtually involve all body systems, but there are two operation centers where most of the information is generated and integrated: the hypothalamus and the gastrointestinal system.

Hypothalamic regulation: from the body weight in the previous decade the principal neurotransmitters involved in the appetite hypothalamic regulation and body energy waste were discovered: Neuropeptide Y (NPY) and agouti-related protein (AgRP) as appetite-stimulating signals (orexigenic signals) and caloric waste decrease inductors; and alpha-melanocyte-stimulating hormone (α-MSH) and cocaine-and-amphetamine-related transcript (CART) as appetite-inhibiting signals (anorexigenic signals) and caloric waste increase inductors.

Two sites whose location and neuroanatomic characteristics allow them to play a critical role in regulating the body weight are the arcuate nucleolus and the median eminence of the hypothalamus. These two hypothalamic nuclei are excluded from the hematoencefalic barrier, which make them accessible to signals travelling in the bloodstream and inform about the energetic and metabolic conditions in the rest of the body.

The neurons from the arcuate nucleus have the higher concentration of leptin receptors (a protein secreted by the adipose tissue which reduces appetite and increases energy waste) in the whole central nervous system (CNS).

Various tissues involved in energetic homeostasis produce hormones having receptors in the neurons of the arcuate nucleus and in the median eminence. Some of these hormones are: insulin coming form the pancreas, Leptin coming from the adipose tissue and Ghrelin coming from the stomach.

The attachment of these hormones to the receptors thereof causes in the neurons of the arcuate nucleus the shooting of a nervous impulse travelling toward the hypothalamic paraventricular nucleus, where afferences are integrated. The axons from the neurons in the paraventricular nucleus go toward the endocrine neurons of the hypothalamus, involved in regulation of the main hormone axes (tirotrope, corticotrope, somatrope, gonadotrope) and toward the neurons from the regulating nucleus of the sympathetic system in the brain stem.

A hormonal a metabolic response is thus generated according to the information from the pancreas, the adipose tissue and the gastrointestinal system. Hunger is a sensation having many psychological and behavioral factors. The substrate for these components of the hunger sensation seems to be in the fibers arising from the paraventricular nucleus to the cerebral cortex.

The hypothalamic interactions taking part on the appetite regulation and energy waste are interesting.

An obese person is that who has a body mass index higher than 30 or more. From the clinical point of view, obesity could be hypertrophic, hyperplasic and morbid.

*Hypertrophic Obesity.* This is peculiar to adults; it is characterized by a great amount of fat in the adipocytes without an increase in the number of fat cells. These individuals are likely to be slim or to maintain an average weight until they are 30 or 40 years old, a time when the weight gain begins. This can be associated with an imbalance between the caloric intake and the usage thereof. People with hypertrophic obesity usually have a central distribution of fat, this problem is likely to be more easily treated.

*Hyperplasic Obesity.* This corresponds to a long-term clinical form wherein the number of adipocytes is higher, as well as the amount of fat contained therein. These individuals are likely to be obese from childhood and to experience a significant weight gain during adolescence. After this age, the number of adipocytes is lifelong maintained. In this obesity form, the fat distribution is central and peripheral. The treatment is considerably more difficult.

*Morbid Obesity.* The term morbid obesity applies to people with more than 100% of its ideal weight. It is so called because it is usually associated with serious and dangerous situations for life such as hypertension, diabetes and atherosclerosis.

This type of obesity increased from 6 to 12 times the mortality risk.

As for the body mass index, in can be said that overweight ranges from 25 to 29.9 kg/m², mild obesity from 30 to 31.9 kg/m², moderated obesity from 32 to 34.9 kg/m², and severe obesity above 35 kg/m².

The main object of a treatment should be the achievement of a healthy and long lasting fitness, to achieve this, it is indispensable to strictly follow the dietetic rules; when starting with a diet, a sudden weight drop is generally observed during the first days due to fat and water loss. Then this weight drop is usually moderated producing a failure in the patient; a certain professional and familiar psychological support must therefore be provided.

5 types of therapeutics can be approached:
1. Food changes.
2. Physical changes.
3. Pharmacotherapy.
4. Psychotherapy.
5. Surgery.

The complementary treatment of a hypocaloric diet, in order to achieve weight loss and the maintenance thereof, is the pharmacotherapy, our interest is to revise the noradrenalin, dopamine and serotonin reuptake inhibitors such as Sibutramine combined with an amino acid such as carnitine.

### SIBUTRAMINE

Sibutramine is rapidly absorbed at the digestive tract, reaching the maximum plasma concentration [Tmax] at 1.2 hours, and undergoes a first-stage extensive hepatic metabolism providing active metabolites M1 (mono-desmetilsibutramina) and M2 (di-desmetil-sibutramina). These metabolites reach the Tmax about the 3 and 4 hours after the administration. Sibutramine and the metabolites M1 and M2 thereof are widely distributed in the body and present a large attachment to human plasmatic proteins (97, 94 and 94%, respectively). Sibutramine is metabolized in the liver, particularly by the cytochrome P450 isoenzyme 3A4 giving rise to metabolites M1 and M2.

These active metabolites are then metabolized by hydroxylation and conjugation giving rise to inactive metabolites M5 and M6. After the administration of indicated Sibutramine therapeutic doses, the following plasma rates are observed: Sibutramine without changes 3%, metabolite M1 6%, M2 12%, M5 52%, and M6 27%. Plasma concentrations of metabolites M1 and M2 reached the stable estate within the four-day treatment and the elimination semi-life thereof is from 14 to 16 hours, respectively.

About 85% of an indicated Sibutramine single dose is expelled through urine and feces within a 15-day period. Most of it (77%) is expelled through urine. The main metabolites in urine are M5 and M6. Sibutramine and active metabolites M1 and M2 are not detected in urine. Food decreases the maximum concentrations of metabolites M1 and M2 and delay their Tmax, but the AUC thereof is not altered. Pharmacological differences clinically significant related to sex or age were not observed.

### CARNITINE

Carnitine is an amino acid used to reduce the adipocytes (fat cells) size. It aids in the weight loss and improves resistance during exercising. It synergically acts with coenzyme Q-10 for increasing cellular energy production (ATP) and thermogenic response to food. The intake of 100 mg of Q-10 plus 500 mg of carnitine from 30 to 60 minutes before meals either with water or juice, fasting or before making the effort (1/2 hours minimum) will make the fats to be used for producing more energy.

In short, the therapeutics employed for treating obesity and overweight is multiple, using pharmaceutical combinations in order to decrease the body mass indexes which are present in patients with obesity or overweight; however, it is our interest to use combinations producing synergy with minimal adverse effects. With these object, tests were made by using the combination Sibutramine-Carnitine, with the aim to assess the synergy, the effectiveness thereof in body mass reduction indexes, as well as the tolerance thereof by evaluating the adverse effects, using fewer dosages of the ingredients.

### BRIEF DESCRIPTION OF THE INVENTION

The present invention describes a pharmaceutical combination comprising a combination of substances that inhibit the reuptake of noradrenalin, dopamine and serotonin and an amino acid for pharmacological use of the composition in the treatment of obesity and overweight; the composition produces a combined product with enhanced properties and a synergistic effect.

The pharmaceutical composition comprises the synergistic combination of a noradrenalin reuptake inhibitor, such as dopamine and serotonin, Sibutramine and or any of the salts thereof and an amino acid known as L-carnitine, Carnitine tartrate and/or any of the salts thereof.

The composition is characterized in that the medicament requiring a fewer dose is L-carnitine, and whose employed salt is the L-carnitine Tartrate.

After multiples trials, it could be proven that the Sibutramine present in a range from 10 to 15 mg per dosage unit provided suitable results.

On the other hand, it could also be proven that the L-carnitine Tartrate, when present in the composition in a range from 300 to 600 mg per dosage unit, provided suitable results.

In a preferred embodiment, the L-carnitine Tartrate is present in a concentration of 400 mg per dosage unit; however, it could also be proven that a concentration of 500 mg per dosage unit is also suitable.

A pharmaceutical composition according to claim 8, wherein the L-carnitine Tartrate is present in a concentration of 600 mg per dosage unit.

As a part of the inventive concept, the use of the combinations of sibutramine and carnitine could be proved to be useful for treating obesity and overweight.

### DETAILED DESCRIPTION OF THE INVENTION

In accordance to the present invention, the pharmacological composition comprising a combination of substances that inhibit the reuptake of noradrenalin, dopamine and serotonin known as Sibutramine combined with an amino acid such as Carnitine was made, whose combination thereof produces a synergistic effect; with a higher reduction in the adipocytes (fat cells) size, and a reduction of caloric intake through the increase of satiety and increase of the energy waste by increasing heat production, and with fewer adverse effects en patients with obesity and/or overweight.

### EXAMPLE 1

A prospective, double-blind, randomized III-phase study was made. 80 individuals were included in the study who fulfilled the inclusion criteria and signed the informed consent letter.

The randomness was stratified in a rate 1:1:1:1 to four treatment groups, Group 1: 20 individuals, mild obesity, BMI 30-31.9 kg/m², Group 2: 20 individuals, moderated obesity, 32-34.9kg/m², Group 3: 20 individuals with severe obesity, BMI above 35 kg/m², and Group 4: Individuals with IMC 27 kg/m², with associated risk factors (10 individuals with diabetes, 6 individuals with arterial hypertension, and 4 individuals with dyslipidemia in the absence of diabetes and hypertension). Individuals with mild, moderated, and severe obesity as well as associated risk factors were included.

Similar treatment regimes were used, whose administration was, orally, a capsule every 24 hours containing Sibutramine 15 mg and Carnitine 600 mg.

### RESULTS

56 patients from the female sex and 24 from the male sex formed part of the study; the average age was of 36.2 years.

The 80 included individuals (100%) satisfactory concluded the clinical investigation study, only two non-serious adverse effects were reported (2.5%) which were mild and transient consisting of abdominal pain and merit neither concomitant therapy nor to suspend the study therapy.

A decrease of BMI from 4% and an average weight loss from 5.3 kg was established. At the end of the treatment.

### CONCLUSIONS

Due to its magnitude and transcendence, it is considered as a Public Health problem in Mexico, as shown by that reported by 2000 Mexican National Health Survey, that from early ages there is a high prevalence of overweight and obesity as a 29% of overweight and obesity is present in adolescents and as to adults and women ranging from 20-59 years old a 36.1% of overweight and 28.1% of obesity was present and in men from the same etarean group 40.9% of overweight and 18.6 of obesity.

Sibutramine/Carnitine showed to be a reliable and effective alternative for reducing BMI, which produces a synergistic effect producing an increase of the satiety effect which the food itself produces, increases heat production; with predictable action in weight loss, and reduces the adipocytes size, maintaining the weigh loss achieved and with an acceptable profile of side effects.

### EXAMPLE 2

A randomized, double-blind and crossover study was made in order to assess the reliability and effectiveness of the combination Sibutramine-Carnitine in patients with obesity and/or overweight suffering from dyslipidemia. 80 individuals were included in the study with a BMI higher than 35 kg/m² diagnosed with severe obesity, with alteration in the profile of lipids which total cholesterol figures was higher than 200 mg/dl.

Combined Sibutramine-carnitine was administered for 4 weeks and subsequently sibutramine alone and carnitine alone for another 4 weeks.

### RESULTS

An statistically significant reduction of BMI was observed when the combination sibutramine-carnitine was administered compared to sibutramine alone, no significant differences were observed when carnitine alone was administered.

As for the lipids profile, a frank progressive reduction from the cholesterol and triglycerides levels were noted compared to those reported during the admission visit, when sibutramine alone was administered; however, when the combination sibutramine-carnitine was employed, a synergistic effect was noted with a faster decrease, with considerably higher drops, and absence of adverse events.

### CONCLUSIONS

Dyslipidemia is a common co-morbid obesity condition. The beneficial changes observed in the lipid profile, when the combination sibutramine-carnitine was employed are evident, combined with the weight loss induced as a consequence of a direct pharmacological effect, results in an excellent therapeutic alternative in patients with obesity and overweight, with excellent tolerance due to the absence of adverse events.

## Claims

1. Synergistic pharmaceutical composition comprising a synergistic combination of a noradrenalin, dopamine and serotonin reuptake inhibitor combined with an amino acid for treating obesity and overweight.

2. The pharmaceutical composition according to claim 1 comprising the synergistic combination, wherein the noradrenalin, dopamine and serotonin reuptake inhibitor and serotonin is Sibutramine and/or any of the salts thereof and an amino acid known as L-carnitine, Carnitine tartrate and/or any of the salts thereof.

3. A pharmaceutical composition according to claim 2, wherein the ingredients combination reduces the body mass index (BMI), reduces the adipocytes (fat cells) size, reduces caloric intake by increasing satiety increases energy waste by increasing heat production, without adverse events.

4. A pharmaceutical composition according to claims 2, 3, and 4, wherein in the medicament requiring a fewer dose is L-carnitine.

5. A pharmaceutical composition according to claim 5, wherein L-carnitine is in the form of L-carnitine Tartrate.

6. A pharmaceutical composition according to claim 2, wherein Sibutramine is present in a range from 10 to 15 mg per dosage unit.

7. A pharmaceutical composition according to claim 2, wherein the L-carnitine Tartrate is present in a range from 300 to 600 mg per dosage unit.

8. A pharmaceutical composition according to claim 8, wherein the L-carnitine Tartrate is present in a concentration of 400 mg per dosage unit.

9. A pharmaceutical composition according to claim 8, wherein the L-carnitine Tartrate is present in a concentration of 500 mg per dosage unit.

10. A pharmaceutical composition according to claim 8, wherein the L-carnitine Tartrate is present in a concentration of 600 mg per dosage unit.

11. The use of the compositions according to claim 2, for treating and controlling obesity and overweight.
